# EUROPEAN PATENT SPECIFICATION

(11) **EP 3 716 863 B1**
(45) Date of publication and mention of the grant of the patent: **27.04.2022**
(21) Application number: 18884592.9
(22) Date of filing: 01.12.2018
(51) Int. Cl.: A61B 17/221, A61B 17/50, A61B 17/00, A61B 17/22

(54) **FOREIGN BODY EXTRACTION DEVICE**
VORRICHTUNG ZUR EXTRAKTION VON FREMDKÖRPERN
DISPOSITIF D'EXTRACTION DE CORPS ÉTRANGER

(30) Priority: 01.12.2017 US 201762593387 P
(43) Date of publication of application: 07.10.2020
(73) Proprietor: Arizona Board of Regents, a body corporate acting on behalf of Arizona State University, Scottsdale, AZ 85257 (US)
(72) Inventor: BLOMMER, Justin, Tempe, Arizona 85281 (US); SMITH, Barbara, Scottsdale, Arizona 85257 (US); SCHMITT, Krystal, Tempe, Arizona 85282 (US); PINA, Christopher, Tempe, Arizona 85282 (US); DRAGER, Erik, Gilbert, Arizona 85295 (US)
(74) Representative: LKGLOBAL Lorenz & Kopf PartG mbB Patentanwälte
(86) International application number: PCT/US2018/063524
(87) International publication number: WO 2019/109060

(56) References cited:
- WO-A2-2010/019776
- US-A- 5 667 525
- US-A- 5 667 525
- US-A1- 2009 222 035
- US-A1- 2012 010 699
- US-A1- 2015 327 876
- US-A1- 2015 327 876
- US-A1- 2016 278 797

## Description

### RELATED APPLICATIONS

This application claims the benefit of US Provisional Patent Application Serial No. 62/593,387, filed on December 1, 2017.

### TECHNICAL FIELD

Aspects of this document relate generally to an extractor device for removing foreign bodies.

### BACKGROUND

The estimated annual rate of occurrence of esophageal food bolus impactions is 13 cases per 100,000 people (GIE). Common causes of food obstructions include eosinophilic esophagitis, strictures of the esophagus, and diffused motor abnormalities of the esophagus. Current limitations in gastrointestinal foreign body extraction devices cause the removal of esophageal food impactions to take 30-90 minutes and can involve upwards of nine devices. Gastroenterologists must remove food bolus impactions from the esophagus through the mouth with an endoscope and extraction devices. Extraction devices often cause shredding of food material, thus requiring multiple passes down the scope and/or multiple extraction attempts, which increases the overall procedure time. This in turn increases the risk of pulmonary aspiration (i.e. material falling into the lungs), a compromised airway, and/or perforation of the esophagus.

US 2015 / 327 876 A1 describes apparatus and methods relating to a medical retrieval device that may include or be used with a catheter. A basket is included with an opening that is oriented transverse to the longitudinal direction in which the catheter extends. The basket may be extended and/or retracted relative to the catheter, and/or may be expanded and/or contracted to, for example, facilitate ensnaring and extraction of a foreign body, object, or polyp, etc. from a patient's esophagus, biliary tree, GI tract, urinary tract, abdominal cavity, kidney, bile duct, urethra, etc.

US 5 667 525 A describes a grasping forceps for an endoscope including a flexible insertion section, an operating wire adapted to pass through the insertion section and to be advanced and retreated in accordance with the operating of an operating section connected with a hand-end side end portion thereof and at least four elastic grasping members of different lengths arranged at the leading end portion of the operating wire and having the habit of flexing such that leading end grasping portions formed at their leading end portions respectively spread outwardly from the central position of the insertion section. At least four elastic grasping members constructing the elastic grasping section are arranged in the order of increasing length to the leading end grasping portions with respect to the leading end face of the insertion section, and between the longest elastic grasping member and the shortest elastic grasping member in length to the leading end grasping portions in the circularly spread state of the elastic grasping members there are provided other elastic grasping members.

US 2012 / 010 699 A1 describes a method and apparatus for facilitating transapical removal of a prosthetic heart valve, i.e., percutaneously implantable valve (PIV), with-out open-heart surgery. The apparatus includes a holding tool for holding the PIV, a cutting tool for separating the PrV from fibrotic tissue accumulating around the PIV, and a removal tool for extracting the PIV from the heart.

Many conventional extraction devices employ prongs extending from the end of a catheter, using hooks to grab onto the food bolus to pull it out. While these devices provide a practitioner with a relatively clear approach to the bolus, they can cause tissue damage, and frequently shredding the bolus and increasing procedure time, risk of pulmonary aspiration, and perforation of the esophagus. Other extraction devices make use of a netted basket that scoops the bolus out of the esophagus. These devices often employ netting to reduce the risk of shredding and possible pulmonary aspiration, but in use require the device to bend around the back of the bolus, a difficult procedure that can easily further impact the bolus, making it more difficult to extract. Additionally, the basket type devices are not very durable even for its one- time use application.

Additionally, conventional extraction devices require the development of proper technique, often requiring the simultaneous operation of a capture mechanism and forward movement toward or past the foreign body; bad timing or too much movement in one of these channel can yield poor results.

### SUMMARY

According to one aspect, an extractor device comprises a handle comprising a drive slide movably coupled to a handle body, the drive slide movable between an extended position and a retracted position, the handle coupled to a first end of a catheter, the drive slide coupled to a first end of a drive wire inside of the catheter, the drive wire substantially equal in length to the catheter. The extractor device further comprises a grasper fixedly coupled to a second end of the drive wire distal to the first end of the drive wire, the grasper comprising four arms of staggered length, each arm having a first end coupled to the drive wire and a second end distal to the drive wire. The second end of each arm comprises an aperture, the grasper movable by the drive slide through the drive wire between a stored position wherein the grasper is located inside the catheter proximate the second end of the catheter, the arms of the grasper collapsed inward and the drive slide in the retracted position, and an open position wherein the grasper is located outside the catheter, the arms extended outward and the drive slide in the extended position. The extractor device also comprises a control ring slideably coupled to the handle body and having a neutral position proximate the first end of the catheter, and a control fiber having a first end and a second end each coupled to the control ring, the control fiber running down the inside of the catheter from the first end of the catheter to the grasper, passing through the aperture at the second end of a first arm of the four arms, the fiber then passing through the aperture of each subsequent arm of the four arms and again through the aperture of the first arm such that a loop is formed between the arms of the grasper when the grasper is in the open position, the control fiber thereafter running up the inside of the catheter from the second end of the catheter to the first end of the catheter. Each arm is curved outward from and biased away from a central axis of the second end of the drive wire. Pulling the control ring away from the neutral position while the grasper is in the open position constricts the loop, deflecting the second ends of the arms towards the central axis and putting the grasper in a capture position, thereby trapping, between the arms, a foreign body received through the loop while the grasper was in the open position. Finally, for each arm of the grasper, the aperture is an eyelet formed from bending the second end of the arm.

Particular embodiments may comprise one or more of the following features. The grasper may further comprise a conical net having an opening for receiving the foreign body and an apex. The loop of control fiber at the second ends of the arms may be woven through the conical net proximate the opening. The apex of the conical net may be proximate the first end of the arms of the grasper. The apex of the net may be between the second end of the catheter and the opening for receiving the foreign body. For each arm of the grasper, the aperture may be formed from bending the second end of the arm inward to form an eyelet. Lastly, the first arm of the four arms of staggered length may be the longest arm.

According to another aspect of the disclosure, an extractor device comprises a handle comprising a drive slide movably coupled to a handle body, the drive slide movable between an extended position and a retracted position, the handle coupled to a first end of a catheter, the drive slide coupled to a first end of a drive wire inside of the catheter, and the drive wire substantially equal in length to the catheter. The extractor device further comprises a grasper fixedly coupled to a second end of the drive wire distal to the first end of the drive wire, the grasper comprising at least three arms of staggered length. Each arm has a first end coupled to the drive wire and a second end distal to the drive wire, the second end of each arm comprising an aperture. The grasper is movable by the drive slide through the drive wire between a stored position wherein the grasper is located inside the catheter proximate the second end of the catheter, the arms of the grasper collapsed inward and the drive slide in the retracted position, and an open position wherein the grasper is located outside the catheter, the arms extended outward and the drive slide in the extended position. The extractor device also comprises a control ring slideably coupled to the handle body and having a neutral position proximate the first end of the catheter, and a control fiber having a first end and a second end, each coupled to the control ring. The control fiber runs down the inside of the catheter from the first end of the catheter to the grasper, passing through the aperture at the second end of a first arm of the at least three arms, the fiber then passing through the aperture of each subsequent arm of the at least three arms and again through the aperture of the first arm such that a loop is formed between the arms of the grasper when the grasper is in the open position, the control fiber thereafter running up the inside of the catheter from the second end of the catheter to the first end of the catheter. Each arm is curved outward from and biased away from a central axis of the second end of the drive wire. Pulling the control ring away from the neutral position while the grasper is in the open position constricts the loop, deflecting the second ends of the arms towards the central axis and putting the grasper in a capture position, thereby trapping, between the arms, a foreign body received through the loop while the grasper was in the open position.

Particular embodiments may comprise one or more of the following features. For each arm of the grasper, the aperture may be an eyelet formed from bending the second end of the arm. For each arm of the grasper, the aperture may be a hole passing through the second end of the arm. The first arm of the at least three arms of staggered length may be the longest arm. Finally, the first arm of the at least three arms of staggered length may be the shortest arm.

According to yet another aspect of the disclosure, an extractor device comprises a handle comprising a drive slide movably coupled to a handle body, the drive slide movable between an extended position and a retracted position, the handle coupled to a first end of a catheter, the drive slide coupled to a first end of a drive wire inside of the catheter, and the drive wire substantially equal in length to the catheter. The extractor device also comprises a grasper fixedly coupled to a second end of the drive wire distal to the first end of the drive wire, the grasper comprising a plurality of arms of staggered length. Each arm has a first end coupled to the drive wire and a second end distal to the drive wire. The grasper is movable by the drive slide through the drive wire between a stored position wherein the grasper is located inside the catheter proximate the second end of the catheter, the arms of the grasper collapsed inward and the drive slide in the retracted position, and an open position wherein the grasper is located outside the catheter, the arms extended outward and the drive slide in the extended position. The extractor device further comprises a control ring slideably coupled to the handle body and having a neutral position proximate the first end of the catheter, and a control fiber having a first end and a second end each coupled to the control ring. The control fiber runs down the inside of the catheter from the first end of the catheter to the grasper, being slideably coupled to the second end of a first arm of the plurality of arms, the fiber also being slideably coupled to the second end of each subsequent arm of the plurality of arms and again to the second end of the first arm such that a loop is formed between the arms of the grasper when the grasper is in the open position, the control fiber thereafter running up the inside of the catheter from the second end of the catheter to the first end of the catheter. Each arm is curved outward from and biased away from a central axis of the second end of the drive wire. Pulling the control ring away from the neutral position while the grasper is in the open position constricts the loop, deflecting the second ends of the arms towards the central axis and putting the grasper in a capture position, thereby trapping, between the arms, a foreign body received through the loop while the grasper was in the open position.

Particular embodiments may be modified or adapted such that, for each arm of the plurality of arms, the second end may comprise an aperture and the control fiber may be slideably coupled to the second end by passing through the aperture. The control fiber may pass through the aperture of the first arm twice.

Aspects and applications of the disclosure presented here are described below in the drawings and detailed description. Unless specifically noted, it is intended that the words and phrases in the specification and the claims be given their plain, ordinary, and accustomed meaning to those of ordinary skill in the applicable arts. The inventors are fully aware that they can be their own lexicographers if desired. The inventors expressly elect, as their own lexicographers, to use only the plain and ordinary meaning of terms in the specification and claims unless they clearly state otherwise and then further, expressly set forth the"special" definition of that term and explain how it differs from the plain and ordinary meaning. Absent such clear statements of intent to apply a"special" definition, it is the inventors' intent and desire that the simple, plain and ordinary meaning to the terms be applied to the interpretation of the specification and claims.

The inventors are also aware of the normal precepts of English grammar. Thus, if a noun, term, or phrase is intended to be further characterized, specified, or narrowed in some way, then such noun, term, or phrase will expressly include additional adjectives, descriptive terms, or other modifiers in accordance with the normal precepts of English grammar. Absent the use of such adjectives, descriptive terms, or modifiers, it is the intent that such nouns, terms, or phrases be given their plain, and ordinary English meaning to those skilled in the applicable arts as set forth above.

The foregoing and other aspects, features, and advantages will be apparent to those artisans of ordinary skill in the art from the DESCRIPTION and DRAWINGS, and from the CLAIMS.

### BRIEF DESCRIPTION OF THE DRAWINGS

The invention will hereinafter be described in conjunction with the appended drawings, where like designations denote like elements, and:
FIG. 1 is a front view of an extractor device;
FIG. 2 is a side view of the extractor device of FIG. 1;
FIG. 3 is a cut-away view of the extractor device of FIG. 1;
FIG. 4 is a side view of a grasper;
FIGs. 5A and 5B are side views of grasper arms;
FIGs. 6A, 6B, and 6C are front views of an extractor device with a grasper in stored, open, and capture positions; and
FIG. 7 is a perspective view of a grasper comprising a net.

### DETAILED DESCRIPTION

This disclosure, its aspects and implementations, are not limited to the specific material types, components, methods, or other examples disclosed herein. Many additional material types, components, methods, and procedures known in the art are contemplated for use with particular implementations from this disclosure. Accordingly, for example, although particular implementations are disclosed, such implementations and implementing components may comprise any components, models, types, materials, versions, quantities, and/or the like as is known in the art for such systems and implementing components, consistent with the intended operation.

The word "exemplary," "example," or various forms thereof are used herein to mean serving as an example, instance, or illustration. Any aspect or design described herein as "exemplary" or as an"example" is not necessarily to be construed as preferred or advantageous over other aspects or designs. Furthermore, examples are provided solely for purposes of clarity and understanding and are not meant to limit or restrict the disclosed subject matter or relevant portions of this disclosure in any manner. It is to be appreciated that a myriad of additional or alternate examples of varying scope could have been presented, but have been omitted for purposes of brevity.

While this disclosure includes a number of embodiments in many different forms, there is shown in the drawings and will herein be described in detail particular embodiments with the understanding that the present disclosure is to be considered as an exemplification of the principles of the disclosed methods and systems, and is not intended to limit the broad aspect of the disclosed concepts to the embodiments illustrated.

The verb "comprise" as is used in this description and in the claims and its conjugations are used in its non-limiting sense to mean that items following the word are included, but items not specifically mentioned are not excluded. In addition, reference to an element by the indefinite article"a" or"an" does not exclude the possibility that more than one of the elements are present, unless the context clearly requires that there is one and only one of the elements. The indefinite article"a" or"an" thus usually means"at least one."

The estimated annual rate of occurrence of esophageal food bolus impactions is 13 cases per 100,000 people (GIE). Common causes of food obstructions include eosinophilic esophagitis, strictures of the esophagus, and diffused motor abnormalities of the esophagus. Current limitations in gastrointestinal foreign body extraction devices cause the removal of esophageal food impactions to take 30-90 minutes and can involve upwards of nine devices. Gastroenterologists must remove food bolus impactions from the esophagus through the mouth with an endoscope and extraction devices. Extraction devices often cause shredding of food material, thus requiring multiple passes down the scope and/or multiple extraction attempts, which increases the overall procedure time. This in turn increases the risk of pulmonary aspiration (i.e. material falling into the lungs), a compromised airway, and/or perforation of the esophagus.

Many conventional extraction devices employ prongs extending from the end of a catheter, using hooks to grab onto the food bolus to pull it out. While these devices provide a practitioner with a relatively clear approach to the bolus, they can cause tissue damage, and frequently shredding the bolus and increasing procedure time, risk of pulmonary aspiration, and perforation of the esophagus. Other extraction devices make use of a netted basket that scoops the bolus out of the esophagus. These devices often employ netting to reduce the risk of shredding and possible pulmonary aspiration, but in use require the device to bend around the back of the bolus, a difficult procedure that can easily further impact the bolus, making it more difficult to extract. Additionally, the basket type devices are not very durable even for its one- time use application.

Additionally, conventional extraction devices require the development of proper technique, often requiring the simultaneous operation of a capture mechanism and forward movement toward or past the foreign body; bad timing or too much movement in one of these channel can yield poor results.

Contemplated herein is an extraction device for the removal of foreign bodies, certain example embodiments of which are illustrated and/or described. The extractor device comprises a handle, a drive slide, and a control ring (or other control structure). For purposes of this disclosure, the terms"extraction device" and"extractor device" are used interchangeably and carry the same meaning. The drive slide is coupled to a grasper via a drive wire, and the control ring is coupled to the grasper via a control fiber. The drive wire and control fiber run through the inside of a catheter coupled to the handle. The grasper comprises a plurality of arms. The control fiber is slideably coupled to the distal ends of the grasper arms to form a loop.

The catheter is inserted into an endoscope and the catheter exits the distal end of the scope. When the drive slide is advanced forward (i.e. toward the catheter), the drive wire is advance further into the catheter, and the grasper is advanced outside the catheter. When the control ring is pushed towards the catheter, the control fiber is advanced further into the catheter, and the loop radius increases. These two actions allow the extraction device to release the material previously secured and/or prepares the grasper to extract material. Once the device surrounds a foreign body, the control ring is pulled, which makes the control fiber retract toward the handle and the loop radius decreases (closes bottom of grasper). The drive slide then can be pulled back (i.e. away from the catheter), which makes the grasper retract toward the inside of the catheter. These two actions allow the device to secure and extract the foreign body.

The contemplated extraction device is able to secure the foreign body for extraction with minimal risk of shredding or slicing through the body or the surrounding tissue. It provides a safer and more efficient method for foreign body extraction, providing procedure time savings and faster procedure room turnover. The device can also reduce average labor costs, duration of anesthesia, and decrease the risk of complications. Additionally, the device provides a practitioner with a clear approach. The operation of the extraction device contemplated herein does not require the degree of skill and technique conventional extraction devices do, yet yields better results.

It should be noted that while much of the following disclosure and use cases are made in the context of the removal of a food bolus from the upper gastrointestinal tract, the extraction devices contemplated herein are not limited to applications in the GI tract. Those skilled in the art will recognize that these devices may be adapted for use in the removal of foreign bodies or tissue from other environments and biological systems.

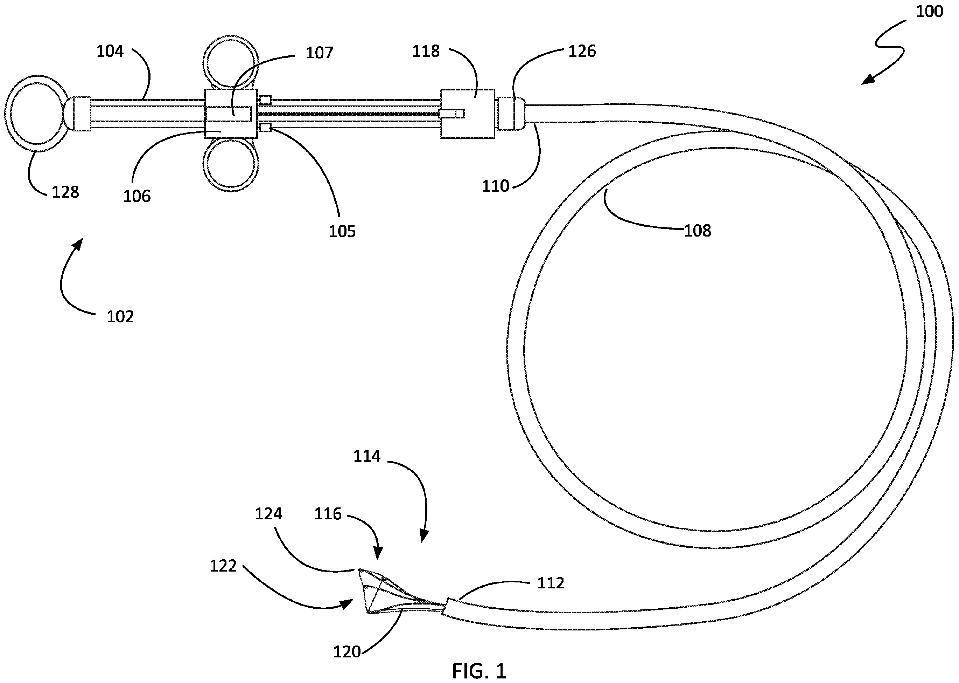

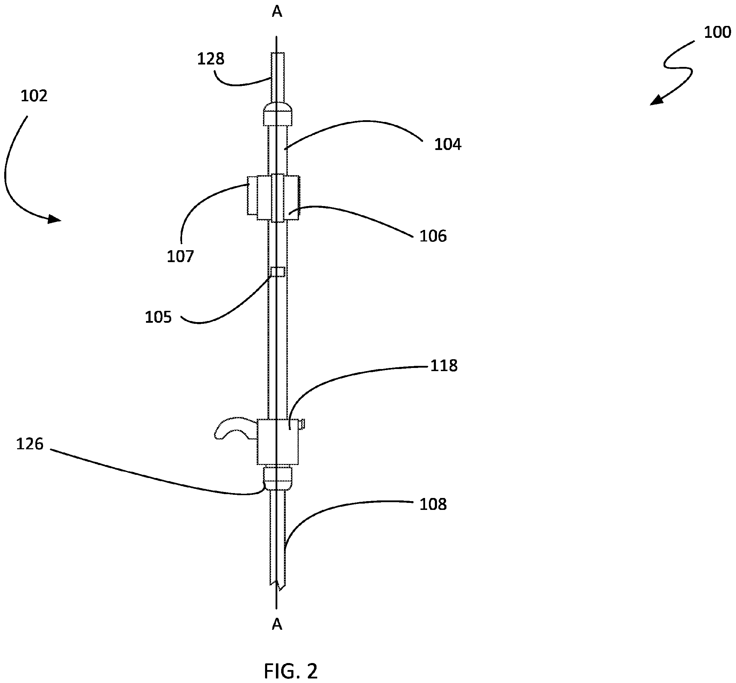

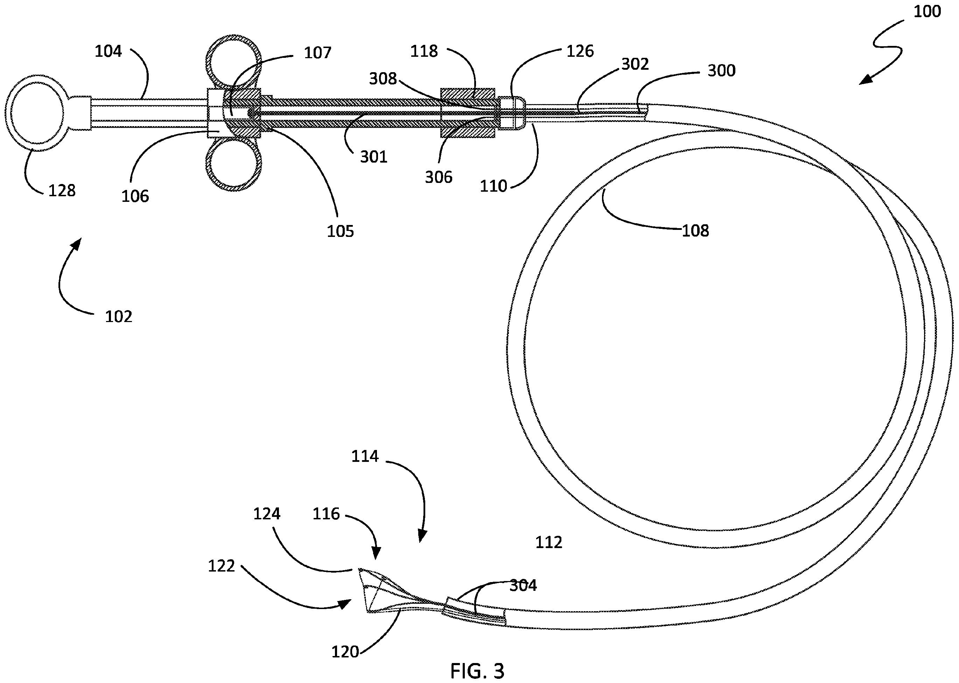

FIGs. 1-3 are front and side views of a non-limiting example of an extractor device 100. Specifically, FIG. 1 is a front view of the extractor device, FIG. 2 is a side view, and FIG. 3 is a cut-away view of the extractor device taken along line A-A of FIG. 2. It should be noted that these Figures are not drawn to scale; relative sizes of elements have been adjusted for clarity. As shown in the example embodiment of FIGs 1-3, the extractor device 100 comprises a handle 102 having a handle body 104, a drive slide 106, a control ring 118, a thumb ring 128, and a cap 126. The drive slide 106 is coupled to a grasper 114 via a drive wire 300, and the control ring 118 is coupled to the grasper 114 via a control fiber (or wire or flexible rod) 120. In certain embodiments, control mechanisms other than control fiber 120 may be employed. The drive wire 300 and control fiber 120 run through the inside of a catheter 108 coupled to the handle. The grasper 114 comprises a plurality of arms 116. The control fiber 120 is slideably coupled to the distal ends of the grasper arms 116 to form a loop 122.

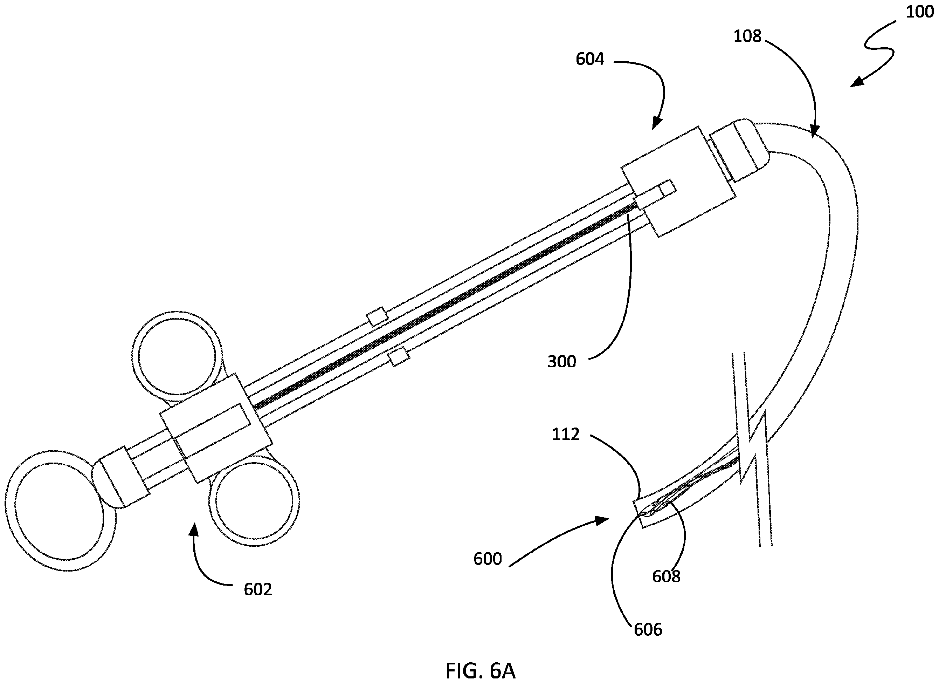

When the drive slide 106 is advanced forward (i.e. toward the catheter 108), the drive wire 300 is advanced further into the catheter, and grasper 114 is advanced outside the other end of the catheter 108. When the control ring 118 is pushed toward the catheter 108, the control fiber 120 is advanced further into the catheter 108, and the loop 122 at the end of the grasper arms 116 opens wider. When the control ring 118 is pulled backward (i.e. away from the catheter), the loop 122 constricts, deflecting the arms 116 inward and trapping a foreign body between the arms 116. The steps for using an extraction device 100 to remove a foreign body will be discussed in greater detail with respect to FIGs. 6A-6C, below.

According to various embodiments, the extraction device 100 is composed of materials making it sterilizable and safe for medical use. In some embodiments, the extraction device 100 may be composed of inexpensive materials allowing it to be a one-time use, disposable item. In other embodiments, such as embodiments configured to operate in more demanding conditions (e.g. smaller space, more arms 116 on the grasper 114, etc.) that require the use of more exotic or expensive materials, the extraction device 100 may be reusable. The materials of various components will be discussed further, below.

It should be noted that in the context of the present description and the claims that follow, the direction "up" or description "above" refers to the direction toward the handle 102, and "down" or "below" refers to the direction toward the grasper 114. For example, the handle 102 is above both the catheter 108 and the grasper 114.

As shown, the handle 102 comprises a handle body 104 with stoppers 105, a drive slide 106 with a drive wire block 107, and a cap 126. The handle body 104 serves as a rail along which the drive slide 106 and the control ring 118 move back and forth, sliding the drive wire 300 and control fiber 120 back and forth inside the catheter 108.

In some embodiments, the handle body 102 may be cylindrical in nature, while in others it may have a non-circular cross-section. The handle body 102 has a void inside for the drive wire 300 and control fiber 120, according to various embodiments, which are actuated, respectively, by the drive slide 106 and control ring 118, which can slide up and down the handle body 102.

The stoppers 105 provide limits to the motion or travel of the drive slide 106 and the control ring 118 along the handle body 104, according to various embodiments. For example, as shown, the drive slide 106 is confined to the portion of handle body 104 below the thumb ring 128 at the top and above the stoppers 105, and the control ring 118 is confined to the portion of the handle body 104 below the stoppers 105 and above the cap 126.

In some embodiments, the handle body 104 may comprise multiple stoppers 105, such as the two stoppers 105 shown in FIG. 1. In other embodiments, the handle body 104 may comprise a single stopper 105 (e.g. a ring protruding from a circumference of the handle body 104, etc.). In still other embodiments, other stopper structures may be employed.

The drive slide 106 is the user's interface with the drive wire 300, and allows them to advance or retract it into/out from the catheter 108, according to various embodiments. As shown, the drive slide 106 may have finger loops. As a specific example, a handle 102 may be configured such that the user inserts their thumb through a thumb ring 128 at the top of the handle 102 and their pointer and middle fingers through loops on the drive slide 106, to operate the drive wire 300 with a single hand.

The grasper 114 of the extraction device 100 gains access to the target foreign body through a catheter 108. The catheter 108 has a first end 110 (i.e. the handle 102 end) and a second end 112 distal to the first end 110 (i.e. the grasper 114 end). The extraction device 100 may make use of a catheter 108 of standard length, such as 200mm or 240mm, or any other length appropriate for the intended application. The diameter of the catheter 108 may range from those compatible with standard-sized endoscopes, such as 2.2mm to 2.6mm, or may be larger, like 4mm or greater.

The grasper 114 is responsible for grabbing the foreign body and securing it well enough that it may be extracted while minimizing the chance for the body to fall apart. As shown, the grasper 114 comprises a plurality of arms 116. According to various embodiments, the arms 116 of the grasper 114 may be staggered in size, facilitating their insertion and travel through the catheter 108. The grasper 114 needs to be able to fit inside a catheter 108 to reach the foreign body, and then open wide enough to capture the foreign body. As a specific example, a grasper 114 may have a closed diameter (e.g. widest part of grasper 114) less than 2.2mm while having an open diameter of 20-28mm.

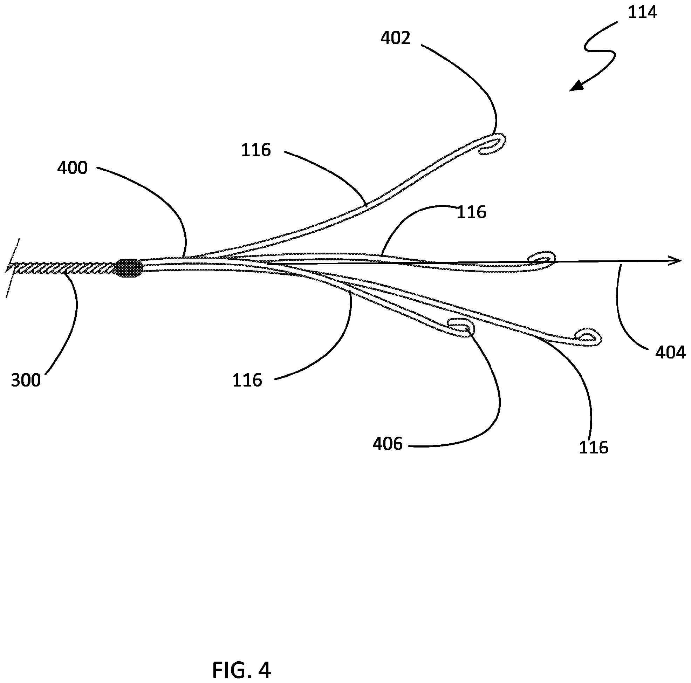

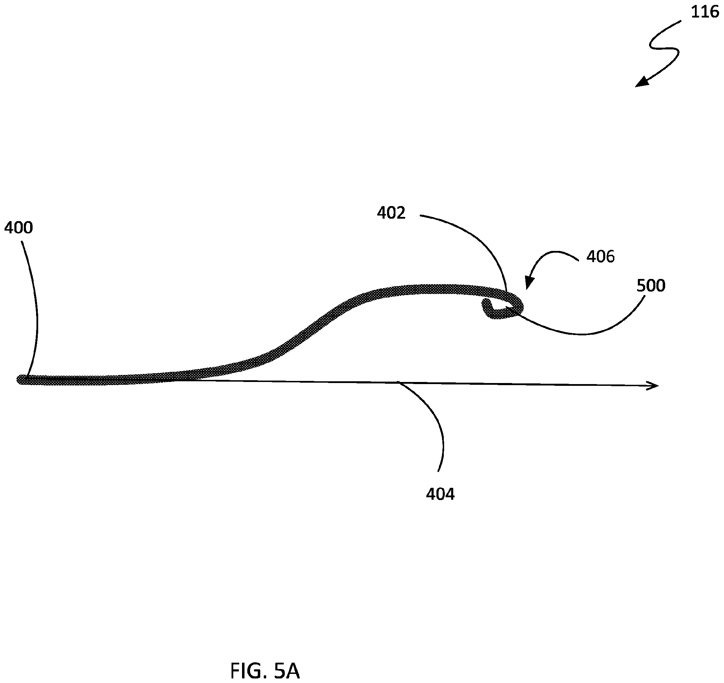

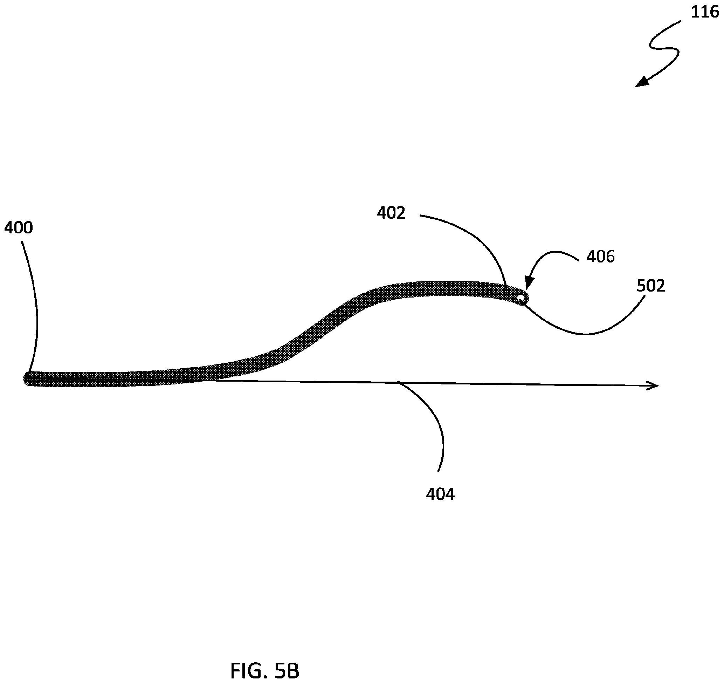

The arms 116 of the grasper 114 need to be rigid enough that they are able to bend to fit inside the catheter 108, spring open when outside the catheter 108, and resist lopsided deformation when the control fiber 120 is pulled and the loop 122 is constricted. According to various embodiments, the arms 116 of the grasper 114 may be constructed of stainless steel (e.g. 302 stainless steel, etc.), or any other flexible material known in the art. The grasper 114 will be discussed in greater detail with respect to FIGs. 4, 5A, and 5B, below.

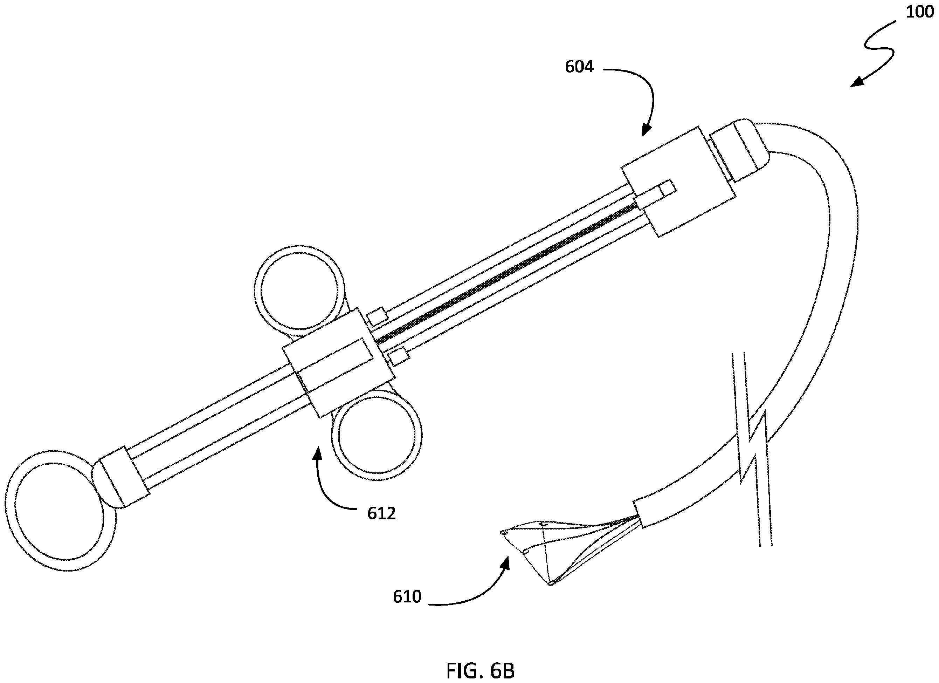

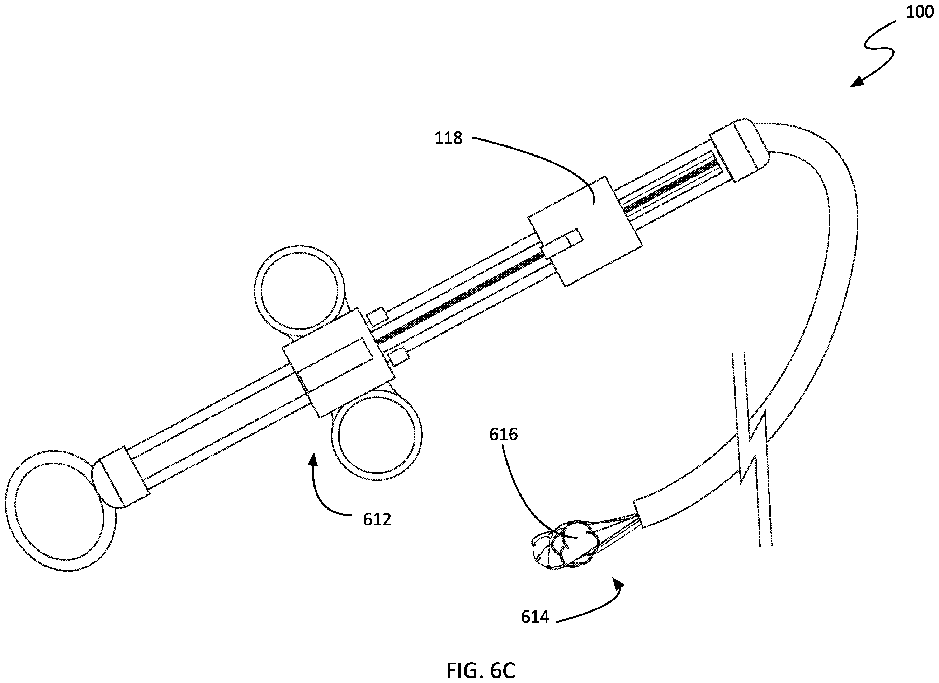

The control ring 118 is the user's interface with the arms 116 of the grasper 114, though the control fiber 120. The control ring 118 is slideably coupled to the handle body 104, and able to move up and down the handle body 104 to advance or retract the control fiber 120 through the catheter 108. In some embodiments, the control ring 118 may comprise a finger hook, to facilitate pulling the control ring 118 upward from a neutral position, which will be discussed in greater detail with respect to FIGs. 6B and 6C.

The control fiber 120 is a flexible filament, having a first end 306 and a second end 308 distal to the first end 306. In some embodiments, including the non-limiting example shown in FIG. 3, both ends of the control fiber 120 may be coupled to the control ring 118. In other embodiments, only one end is coupled to the control ring 118, while the other end of the control fiber 120 is affixed to the handle 102 or grasper 114 such that it does not move. In embodiments where both ends of the control fiber 120 are coupled to the control ring 118, the control fiber 120 may pass through the catheter 108 twice, while in other embodiments, it may pass through the catheter one or more times.

The control fiber is slideably coupled to the second or lower end of each arm 116 of the grasper 114. In the context of the present description and the claims that follow, the control fiber being slideably coupled to the arms means that the relative position of "fiber" with respect to each arm is fixed (i.e. the fiber is always located at a specific location on each arm) while the fiber itself is able to move through or past the arms. In some embodiments, each arm 116 comprises an aperture through which the fiber passes, while in others the fiber 120 may reside within a notch in the arm. Different types of aperture will be discussed with respect to FIG. 4, below.

As shown, the control fiber 120 is slideably coupled to the lower end of each arm 116 of the grasper 114, starting with a first arm 124, and then coupling to each subsequent, or neighboring, arm 116 until once again coupling to the first arm 124 to form a loop at the ends of the arms 116. In some embodiments, the control fiber 120 crosses itself at the first arm 124, while in other embodiments, the control fiber 120 may follow two parallel paths with respect to the first arm 124.

When the control ring 118 is pulled upward, the loop 122 is constricted and the lower ends of the arms is pulled inward. Any material or body located between the arms is then trapped, and can be pulled along with the rest of the device for extraction. The first arm will be discussed further with respect to FIG. 6A, below.

The foreign body being extracted passes through the loop 122, therefore making the size of the loop 122 an important design consideration for various embodiments of the extraction device 100. The available loop size is in part dictated by the size and shape of the arms 116 of the grasper 114. The length of the arms 114 is dictated in part by the available travel of the drive slide 106 (e.g. how much the drive wire 300 can move through the catheter). According to various embodiments, the travel of the drive slide 106 is dictated in part by the size of a practitioner's hand. For the drive slide 106 to be operated with a single hand, which is necessary to be able to operate the control ring 118 at the same time, it must be within a comfortable range. In some embodiments, the drive slide range may be between 70mm and 100 mm. In a specific embodiment, the drive slide travel is between 70mm and 90mm, and yields a loop circumference of 20mm-28mm.

According to various embodiments, the loop 122 is relatively taut when the grasper 114 is open and ready to capture a foreign body. The tension in the control fiber 120 should be high enough that the loop 122 can be pushed past the foreign body, but loose enough that it can move around the irregular shape of a foreign body, rather than slicing through it. The tension of the control fiber 120 is dictated by the position of the control ring 118 along its available range, or travel, on the handle body 104. In another embodiment, the travel of the control ring 118 is limited to 50mm and the drive slide 106 limited to 80mm.

The control fiber 120 is composed of a strong yet flexible material able to easily slide past the arms 116 of the grasper 114 while withstanding the strain exerted by the arms 116 while gripping a foreign body. In some embodiments, the control fiber 120 is composed of polypropylene, while in others it is composed of nylon, or other polymers known in the art. In some embodiments, the control fiber 120 may be a suture. In some embodiments, the diameter of the control fiber 120 may range from 0.8 to 1.2 mm, while in others it can 0.2mm or larger. In some embodiments the control fiber 120 is a single strand, while in others it may be braided or twisted, as is known in the art.

The drive wire 300 connects the drive slide 106 to the grasper 114, and comprises a first end 302 (e.g. the upper end) and a second end 304 (e.g. the lower end). It is substantially equal in length to the catheter 108 through which it passes, such that when the drive slide 106 is at its highest position, the grasper 114 is retracted inside the catheter, and when the drive slide 106 is at its lowest position, the grasper is outside of the catheter 108 and ready to receive a foreign body for extraction.

The drive wire 300 is coupled to the grasper 114. In some embodiments, the arms 116 of the grasper 114 are directly affixed to the second end 304 of the drive wire 300. For example, in one embodiment, the arms 116 are laser welded on to the second end 304 of the drive wire 300. In other embodiments, the grasper 114 may be an already-assembled unit before being attached to the end of the drive wire 300. In some embodiments, the grasper 114 may be releasably coupled to the drive wire 300, making it replaceable, which may be advantageous in embodiments that make use of exotic materials.

The drive wire 300 is composed of resilient material strong enough to endure the strains of pulling on a foreign body, and flexible enough to bend with the catheter to reach the extraction site. In some embodiments, the drive wire 300 is made of stainless steel, while in others it may be made of other materials known in the art. The drive wire 300 needs to be small enough to fit inside the catheter. In one embodiment, the drive wire is 1.59mm in diameter, while in another it is 0.79mm in diameter. The drive wire 300 may be a single strand, or it may be multiple strands that are braided or twisted.

According to various embodiments, the drive wire 300 is attached to the drive slide 106 through a handle wire 301, which is a segment of material that is much more rigid than the drive wire 300, and is predominantly inside of the handle 102. As a specific example, a drive wire 300 may be welded onto a handle wire 301 made of 16-gauge stainless steel, which is then attached to the drive slide 106. The use of a handle wire 301 is known in the catheter arts.

According to various embodiments, the drive wire 300 may be attached to the drive slide 106 through a drive slide block 107. As shown, the drive slide 106 may have an opening in its side, which is aligned with a track running the length of the handle body 104. The drive slide block 107 fits inside the opening and the track, and is coupled to the drive wire 300 or handle wire 301, keeping the drive slide 106 slideably coupled to the handle body 104 and fixedly coupled to the drive wire 300.

In a specific embodiment, the handle 102 may be assembled as follows. The drive slide 106 may be slide onto the upper side of the handle body 104. The drive slide block is inserted through the opening in the side of the drive slide 106. The handle wire 301 is inserted through a hole in the bottom of the block, and glued to the block 107. The thumb ring 128 is attached to the top of the handle body 104. The control ring 118 is slid onto the lower end of the handle body 104, and confined by the attachment of the cap 126 to the lower end of the handle body 104, the handle wire 301 passing through a hole in the cap. The control fiber 120 is then passed through the hole in the cap and attached to the control ring 118.

FIG. 4 is a side view of a nondimiting example of a grasper 114, without a control fiber 120. As shown, the grasper 114 comprises a plurality of arms 116, each having a first end 400 (e.g. upper end) and a second end 402 (e.g. lower end). In some embodiments, the grasper 114 may have four arms 116, while in others it may have more than 3 (e.g. to be able to create a loop 122), such as five arms 116, six arms 116, or more.

In some embodiments, the arms 116 of the grasper 114 may be identical, while in others they may have different lengths. The use of arms 116 having staggered lengths is advantageous in embodiments where the arms 116 have an aperture 406 at the second end 402. Staggering the lengths facilitates fitting the grasper 114 inside the catheter 108 such that the second ends 402 are not pushed up against each other making the grasper 114 wider than needed.

In some embodiments, the control fiber 120 may slideably couple to the arms 116 by passing through an aperture 406 at the second end 402. According to various embodiments, the aperture 406 is ideally no larger than necessary to allow the control fiber 120 to pass through with minimal resistance. Such a restriction may maximize the overall capturing capacity of the grasper 114.

In some embodiments, the apertures 406 may all be the same size, while in other embodiments, the aperture 406 of the first arm 124 may be larger, as the control fiber 120 passes through that aperture twice. In some embodiments, the arms 116 may be identical, while in others the aperture 406 of the first arm 124 may have a different orientation. As a specific example, the aperture 406 of the first arm 124 may be tangential to the loop 122, and the apertures 406 of the remaining arms 116 may be perpendicular to the loop 122, to facilitate the sliding of the fiber 120. Further variations in apertures 406 will be discussed further with respect to FIGs. 5A and 5B.

FIG. 4 also shows a central axis 404 of the second end 304 of the drive wire 300, which serves as a point of reference in the following discussion of arm geometry.

FIGs. 5A and 5B are side views of non-limiting examples of grasper arms 116. Specifically, FIG. 5A shows a side view of an arm 116 whose aperture 406 is an eyelet 500, and FIG. 5B shows a side view of an arm 116 whose aperture 406 is a hole 502. These two exemplary apertures 406 are not meant to be limiting, as other means for slideably coupling a control fiber 120 the second end 402 of an arm 116 have been discussed elsewhere in this disclosure, and still others are known in the art.

According to various embodiments, the arms 116 of a grasper 114 each bend outward from the central axis 404, and are biased away from the central axis 404, such that as they are pushed forward by the drive wire 300 to exit the catheter, they spring into, are drive toward, or biased toward, and open position 610, as will be discussed with respect to FIG. 6B, below. The bias of the arms 116 may be created by shaping them before coupling to each other or to the drive wire 300, as is known in the art.

As shown, in some embodiments, the arms 116 bend outward from the central axis 404, yet the portion of the arm 114 near the second end 402 is concave inward. Such as shape facilitates the constriction of the control fiber loop 122, which brings the second ends 402 of the arms inward to trap a foreign body and put the grasper 114 into a capture position 614, which will be discussed in greater detail with respect to FIG. 6C.

In some embodiments, the angle formed between the first end 400 and second end 402 of an arm 116 while the grasper 114 is in the open position 610 is between 30 and 60 degrees. In some embodiments, the arm 116 may bend outward from the central axis 404 in a smooth curve, while in others it may have one or more vertex. As previously discussed, the length of arms 116 may, at least in part, depend upon the anticipated hand size (for manual operation) of an average practitioner or travel length of the drive slide 106. As a specific example, in one embodiment, the arms 116 may be cylindrical, with an average length of 38mm and a diameter of 0.5mm.

The arms 116 are composed of materials that are flexible yet stiff enough to have a strong enough bias to spring open and create the control fiber loop 122 when pushed out of the catheter, as well as withstand the strain of being bent around a foreign body to capture it for extraction. In some embodiments, the arms 116 may be composed of stainless steel (e.g. 302 stainless steel), while in others they may be composed of other materials known in the art.

FIG. 5 A shows an arm 116 whose aperture 406 is an eyelet 500 that is formed by bending the second end 402 of the arm 116 to create a circle. In some embodiments, the bend is inward, towards the central axis 404, while in others it may be in a different direction. In some embodiments, the eyelet 500 may be sealed closed (e.g. laser welding or spot welding the end 402 to the arm 116, etc.), while in others the end may be bent until any gap between the end 402 and the arm 116 is smaller than the fiber 120. As an option, the interior and edges of the eyelet 500 may be machined or otherwise made smooth, to reduce friction and facilitate sliding the control fiber 120 through the eyelet 500.

FIG. 5B shows an arm 116 whose aperture 406 is a hole 502 that passes through the second end 402 of the arm 116. In some embodiments, the hole 502 may be linear, while in other embodiments, the hole 502 may follow the path of the loop 122 or an idealized loop 122. In some embodiments, the hole 502 may be formed by machining or casting, while in others the hole 502 may be cut with a laser, or other cutting methods known in the art. As an option, the interior and edges of the hole 502 may be machined or otherwise made smooth, to reduce friction and facilitate sliding the control fiber 120 through the hole 502.

FIGs. 6A-6C show a front view of non-limiting example of an extractor device 100 in various stages of operation. FIG. 6 A shows a front view of an extractor device 100, whose drive slide 106 is in a retracted position 602. In the context of the present description and the claims that follow, the retracted position 602 of the drive slide 106 is the position along the handle body 104 at which the drive wire 300 is pulled back enough that the grasper 114 is entirely inside of the second end 112 of the catheter 108. Putting the drive slide 106 into the retracted position 602 places the grasper 114 into a stored position 600, as shown. In such a configuration, the position of the control ring 118 may be of no import.

According to various embodiments, the configuration shown in FIG. 6A, where the drive slide 106 is in the retracted position 602 and the grasper 114 is in the stored position 600 is the initial configuration for using the extraction device. After being placed in this configuration, the catheter 108 is inserted into an endoscope and then exits the distal end of the endoscope (not shown), in preparation for insertion.

As shown, the arms 116 may be of staggered length, to make the most of the narrow space available inside the catheter 108. In some embodiments, the first arm 124 (i.e. the arm 116 where the loop 122 begins and ends) is the longest arm 606, while in others it is the shortest arm 608, and in still others it is neither the longest 606 or shortest 608.

FIG. 6B shows a front view of the extractor device 100 of FIG. 6 A, whose drive slide 106 is in an extended position 612. This configuration may be arrived at once the second end of the catheter is near the foreign body that is to be extracted. In the context of the present description and the claims that follow, the extended position 612 of the drive slide 106 is the position along the handle body 104 at which the drive wire 300 is pushed forward enough that the grasper 114is substantially outside of the second end 112 of the catheter 108. Putting the drive slide 106 into the extended position 612 places the grasper 114 into an open position 610, as shown. In such a configuration, the position of the control ring 118 is in a neutral position 604, allowing the loop 122 to form and establishing a sufficient level of tension in the control fiber 120, as previously discussed.

Finally, FIG. 6C shows a front view of the extractor device 100 of FIGs. 6A and 6B, whose control ring 118 has been pulled away from the neutral position 604, pulling some of the control fiber 120 into the catheter 108 and constricting the loop 122 (e.g. reducing loop radius, reducing loop circumference, etc.), thereby bending or deflecting the second ends 402 of the arms 116 inward and putting the grasper 114 into a capture position 614. This configuration may be arrived at once the foreign body 616 is inside the "capture volume" of the grasper 114 (e.g. is in between the arms, etc.).

In the context of the present description and the claims that follow, the capture position 614 of the grasper 114 is the position where the second ends 402 are closer to the central axis 404 than they are while in the open position 610. Any foreign body 616 that was between the arms 116 and near the central axis 404 before the grasper 114 was placed in the capture position 614 will be trapped and ready for extraction by pulling the entire device out of the patient.

According to various embodiments, the capture position 614 is maintained by maintaining pressure on the control ring 118, upward. The practitioner can keep tension on the control fiber 120 by pulling upward on the control ring 118 with one hand while pulling the entire device out of the patient's mouth with the other. Again, it should be noted that while this example is made in the context of the extraction of a food bolus from a gastrointestinal tract, the devices contemplated herein may be applied in other contexts as well.

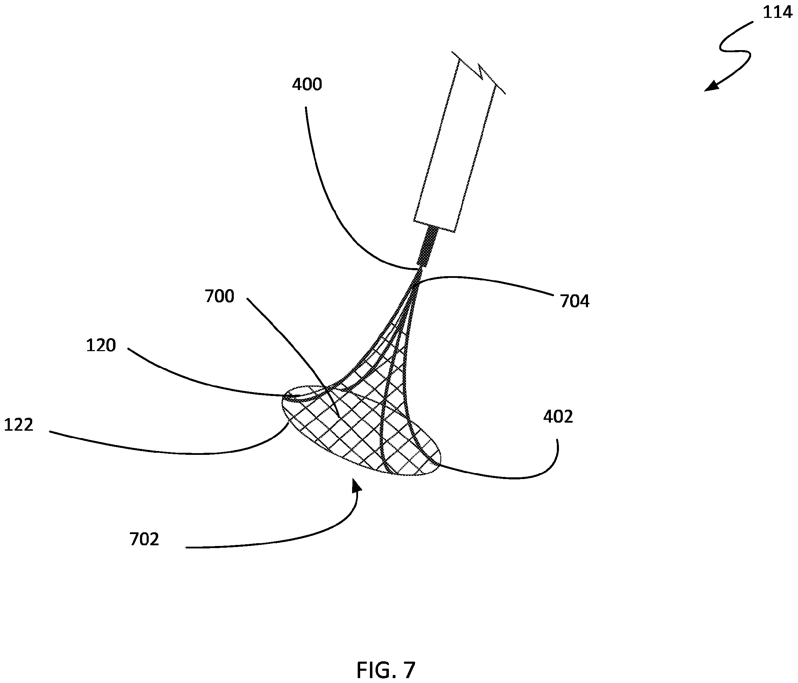

FIG. 7 is a perspective view of a grasper 114 comprising a conical net 700. The addition of a net 700 to the grasper 114 may enhance the extraction device's 100 ability to prevent aspiration of material into the lungs by containing any "crumbs" that otherwise would fall out into the airway. This has the advantage of reducing or eliminating the need for using an endotracheal tube or overtube to protect the airway, reducing cost, time needed, and the chance of procedural complications. However, the use of a net 700 may come at a cost, as it increases the amount of material that needs to fit inside of the catheter 108.

As shown, the net 700 has an opening 702 for receiving the foreign body 616, and an apex 704 that is distal to the opening 702 and proximate the first end of the grasper arms 116 (or second end 304 of the drive wire 300). In some embodiments, the net 700 may be couple to the arms 116 of the grasper 114. As an option, the control fiber 120 may be woven through the net 700 along the loop 122, proximate the opening 702. In some embodiments, the apex 704 may also be coupled to the grasper 114.

Unlike conventional extraction devices making use of a net, the net 700 of the grasper 114 shown in FIG. 7 is pointing downward, toward the foreign body 616. This means that the complicated bending maneuver required by conventional netted extraction devices to scoop up the body is not needed. The benefits of using a net of the kind disclosed herein are present, with fewer of the downsides found in conventional extractors using nets.

In certain embodiments, an extractor device comprises a handle comprising a drive slide movably coupled to a handle body, the drive slide movable between an extended position and a retracted position, the handle coupled to a first end of a catheter, the drive slide coupled to a first end of a drive wire inside of the catheter, the drive wire substantially equal in length to the catheter. The extractor device further comprises a grasper fixedly coupled to a second end of the drive wire distal to the first end of the drive wire, the grasper comprising four arms of staggered length, each arm having a first end coupled to the drive wire and a second end distal to the drive wire. The second end of each arm comprises an aperture, the grasper movable by the drive slide through the drive wire between a stored position wherein the grasper is located inside the catheter proximate the second end of the catheter, the arms of the grasper collapsed inward and the drive slide in the retracted position, and an open position wherein the grasper is located outside the catheter, the arms extended outward and the drive slide in the extended position. The extractor device also comprises a control ring slideably coupled to the handle body and having a neutral position proximate the first end of the catheter, and a control fiber having a first end and a second end each coupled to the control ring, the control fiber running down the inside of the catheter from the first end of the catheter to the grasper, passing through the aperture at the second end of a first arm of the four arms, the fiber then passing through the aperture of each subsequent arm of the four arms and again through the aperture of the first arm such that a loop is formed between the arms of the grasper when the grasper is in the open position, the control fiber thereafter running up the inside of the catheter from the second end of the catheter to the first end of the catheter. Each arm is curved outward from and biased away from a central axis of the second end of the drive wire. Pulling the control ring away from the neutral position while the grasper is in the open position constricts the loop, deflecting the second ends of the arms towards the central axis and putting the grasper in a capture position, thereby trapping, between the arms, a foreign body received through the loop while the grasper was in the open position. Finally, for each arm of the grasper, the aperture is an eyelet formed from bending the second end of the arm.

Particular embodiments may comprise one or more of the following features. The grasper may further comprise a conical net having an opening for receiving the foreign body and an apex. The loop of control fiber at the second ends of the arms may be woven through the conical net proximate the opening. The apex of the conical net may be proximate the first end of the arms of the grasper. The apex of the net may be between the second end of the catheter and the opening for receiving the foreign body. For each arm of the grasper, the aperture may be formed from bending the second end of the arm inward to form an eyelet. Lastly, the first arm of the four arms of staggered length may be the longest arm.

According to other embodiments, an extractor device comprises a handle comprising a drive slide movably coupled to a handle body, the drive slide movable between an extended position and a retracted position, the handle coupled to a first end of a catheter, the drive slide coupled to a first end of a drive wire inside of the catheter, and the drive wire substantially equal in length to the catheter. The extractor device further comprises a grasper fixedly coupled to a second end of the drive wire distal to the first end of the drive wire, the grasper comprising at least three arms of staggered length. Each arm has a first end coupled to the drive wire and a second end distal to the drive wire, the second end of each arm comprising an aperture. The grasper is movable by the drive slide through the drive wire between a stored position wherein the grasper is located inside the catheter proximate the second end of the catheter, the arms of the grasper collapsed inward and the drive slide in the retracted position, and an open position wherein the grasper is located outside the catheter, the arms extended outward and the drive slide in the extended position. The extractor device also comprises a control ring slideably coupled to the handle body and having a neutral position proximate the first end of the catheter, and a control fiber having a first end and a second end, each coupled to the control ring. The control fiber runs down the inside of the catheter from the first end of the catheter to the grasper, passing through the aperture at the second end of a first arm of the at least three arms, the fiber then passing through the aperture of each subsequent arm of the at least three arms and again through the aperture of the first arm such that a loop is formed between the arms of the grasper when the grasper is in the open position, the control fiber thereafter running up the inside of the catheter from the second end of the catheter to the first end of the catheter. Each arm is curved outward from and biased away from a central axis of the second end of the drive wire. Pulling the control ring away from the neutral position while the grasper is in the open position constricts the loop, deflecting the second ends of the arms towards the central axis and putting the grasper in a capture position, thereby trapping, between the arms, a foreign body received through the loop while the grasper was in the open position.

Particular embodiments may comprise one or more of the following features. For each arm of the grasper, the aperture may be an eyelet formed from bending the second end of the arm. For each arm of the grasper, the aperture may be a hole passing through the second end of the arm. The first arm of the at least three arms of staggered length may be the longest arm. Finally, the first arm of the at least three arms of staggered length may be the shortest arm.

According to still other embodiments, an extractor device comprises a handle comprising a drive slide movably coupled to a handle body, the drive slide movable between an extended position and a retracted position, the handle coupled to a first end of a catheter, the drive slide coupled to a first end of a drive wire inside of the catheter, and the drive wire substantially equal in length to the catheter. The extractor device also comprises a grasper fixedly coupled to a second end of the drive wire distal to the first end of the drive wire, the grasper comprising a plurality of arms of staggered length. Each arm has a first end coupled to the drive wire and a second end distal to the drive wire. The grasper is movable by the drive slide through the drive wire between a stored position wherein the grasper is located inside the catheter proximate the second end of the catheter, the arms of the grasper collapsed inward and the drive slide in the retracted position, and an open position wherein the grasper is located outside the catheter, the arms extended outward and the drive slide in the extended position. The extractor device further comprises a control ring slideably coupled to the handle body and having a neutral position proximate the first end of the catheter, and a control fiber having a first end and a second end each coupled to the control ring. The control fiber runs down the inside of the catheter from the first end of the catheter to the grasper, being slideably coupled to the second end of a first arm of the plurality of arms, the fiber also being slideably coupled to the second end of each subsequent arm of the plurality of arms and again to the second end of the first arm such that a loop is formed between the arms of the grasper when the grasper is in the open position, the control fiber thereafter running up the inside of the catheter from the second end of the catheter to the first end of the catheter. Each arm is curved outward from and biased away from a central axis of the second end of the drive wire. Pulling the control ring away from the neutral position while the grasper is in the open position constricts the loop, deflecting the second ends of the arms towards the central axis and putting the grasper in a capture position, thereby trapping, between the arms, a foreign body received through the loop while the grasper was in the open position.

Particular embodiments may be modified or adapted such that, for each arm of the plurality of arms, the second end may comprise an aperture and the control fiber may be slideably coupled to the second end by passing through the aperture. The control fiber may pass through the aperture of the first arm twice.

Where the above examples, embodiments and implementations reference examples, it should be understood by those of ordinary skill in the art that other extraction devices and systems and manufacturing devices and examples could be intermixed or substituted with those provided. In places where the description above refers to particular embodiments of an extraction device and customization methods, it should be readily apparent that a number of modifications may be made and that these embodiments and implementations may be applied to other extraction device customization technologies as well. Accordingly, the disclosed subject matter is intended to embrace all such alterations, modifications and variations that fall within the scope of the disclosure and the knowledge of one of ordinary skill in the art.

Any dimensions presented in this document are for example only and not a limitation on the scope of this disclosure. It will be understood that embodiments are not limited to the specific components disclosed herein, as virtually any components consistent with the intended operation of the method or system may be utilized. Accordingly, for example, although particular materials, structures, and couplings may be disclosed, such components may comprise any shape, size, style, type, model, version, class, grade, measurement, concentration, material, weight, quantity, or the like consistent with the intended operation of an extraction device.

Accordingly, the components defining any extraction device embodiment may be formed of any of many different types of materials or combinations thereof that can readily be formed into shaped objects provided that the components selected are consistent with the intended operation of an extraction device embodiment. For example, the components can comprise one or more: polymers such as thermoplastics (such as ABS, Fluoropolymers, Polyacetal, Polyamide; Polycarbonate, Polyethylene, Polysulfone, or other similar material), thermosets (such as Epoxy, Phenolic Resin, Polyimide, Polyurethane, Silicone, or other similar material); glasses (such as quartz glass), carbon-fiber, aramid-fiber, any combination thereof, or other similar material; composites; metals, such as zinc, nitinol, magnesium, titanium, copper, lead, iron, steel, carbon steel, alloy steel, tool steel, stainless steel, brass, tin, antimony, pure aluminum, 1100 aluminum, aluminum alloy, or other similar materials; alloys, such as aluminum alloy, titanium alloy, magnesium alloy, copper alloy, any combination thereof, or other similar materials; and one or more of any of the above with one or more or other similar material.

Various extraction device embodiments may be manufactured using conventional procedures as added to and improved upon through the procedures described here. Some components may be manufactured simultaneously and integrally joined with one another, while other components may be purchased pre-manufactured or manufactured separately and then assembled with the integral components.

Accordingly, manufacture of these components separately or simultaneously may involve one or more of extrusion, pultrusion, vacuum forming, injection molding, blow molding, resin transfer molding, casting, forging, cold rolling, milling, drilling, reaming, turning, grinding, stamping, cutting, bending, welding, soldering, hardening, riveting, punching, plating, or other similar process. If any of the components are manufactured separately, they may then be coupled with one another in any suitable manner, such as with adhesive, a weld, a fastener ( e.g ., a bolt, a nut, a screw, a nail, a rivet, a pin), wiring, any combination thereof, and/or the like for example, depending on, among other considerations, the particular material forming the components. Upon reading the teachings of this specification, those with ordinary skill in the art will appreciate that, under certain circumstances, considering issues such as changes in technology, user requirements, etc ., a variety of fastening devices may be used to affix, couple, or releasably couple, (as those words are used herein) one or more components of the present disclosure. These fastening devices may comprise one or more of the following: adhesives, belts, bolts, buckles, clasps, latches, locks, screws, snaps, clamps, connectors, couplings, ties, or other fastening means yet to be developed.

Likewise, upon reading the teachings of this specification, those with ordinary skill in the art will appreciate that, under certain circumstances, considering issues such as changes in technology, subject requirements, etc ., a variety of fastening devices, such as adhesives, belts, bolts, buckles, clasps, latches, locks, screws, snaps, clamps, connectors, couplings, ties or other fastening means yet to be developed may be used in lieu of - or in conjunction with - any of the fasteners or fastening means discussed above.

It will be understood that the assembly of extraction device embodiments are not limited to the specific order of steps as disclosed in this document. Any steps or sequence of steps of the assembly of extraction device embodiments indicated herein are given as examples of possible steps or sequence of steps and not as limitations, since various assembly processes and sequences of steps may be used to assemble extraction device embodiments.

In places where the description above refers to particular embodiments, it should be readily apparent that a number of modifications may be made and that these embodiments may be applied to other embodiments disclosed or undisclosed. The presently disclosed embodiments are, therefore, to be considered in all respects as illustrative and not restrictive, the scope of the invention being defined by the appended claims rather than the foregoing description.

## Claims

1. An extractor device (100), comprising:
a handle comprising a drive slide (106) movably coupled to a handle body, the drive slide (106) movable between an extended position and a retracted position, the handle coupled to a first end of a catheter (108), the drive slide (106) coupled to a first end of a drive wire (300) inside of the catheter (108), the drive wire (300) substantially equal in length to the catheter (108);
a grasper (114) fixedly coupled to a second end of the drive wire (300) distal to the first end of the drive wire (300), the grasper (114) comprising a plurality of arms of staggered length, each arm having a first end coupled to the drive wire (300) and a second end distal to the drive wire (300), the grasper (114) movable by the drive slide (106) through the drive wire (300) between a stored position wherein the grasper (114) is located inside the catheter (108) proximate the second end of the catheter (108), the arms of the grasper (114) collapsed inward and the drive slide (106) in the retracted position, and an open position wherein the grasper (114) is located outside the catheter (108), the arms extended outward and the drive slide (106) in the extended position;
a control ring (118) slideably coupled to the handle body and having a neutral position proximate the first end of the catheter (108); and
a control fiber (120) having a first end and a second end each coupled to the control ring (118), the control fiber (120) running down the inside of the catheter (108) from the first end of the catheter (108) to the grasper (114), being slideably coupled to the second end of a first arm of the plurality of arms, the fiber also being slideably coupled to the second end of each subsequent arm of the plurality of arms and again to the second end of the first arm such that a loop is formed between the arms of the grasper (114) when the grasper (114) is in the open position, the control fiber (120) thereafter running up the inside of the catheter (108) from the second end of the catheter (108) to the first end of the catheter (108);
wherein each arm is curved outward from and biased away from a central axis of the second end of the drive wire (300);
wherein pulling the control ring (118) away from the neutral position while the grasper (114) is in the open position constricts the loop, deflecting the second ends of the arms towards the central axis and putting the grasper (114) in a capture position, thereby trapping, between the arms, a foreign body received through the loop while the grasper (114) was in the open position.

2. The extractor device according to claim 1, the plurality of arms being four arms, the second end of each arm comprising an aperture;
wherein the slidable coupling of the control fiber (120) to the second end of the first arm of the plurality of arms and to the second end of each subsequent arm of the plurality of arms and again to the second end of the first arm is achieved by the control fiber (120) passing through the corresponding aperture at the corresponding second end of the corresponding arm; and
wherein, for each arm of the grasper (114), the aperture is an eyelet formed from bending the second end of the arm.

3. The extraction device of claim 2, the grasper (114) further comprising a conical net having an opening for receiving the foreign body and an apex, the loop of control fiber (120) at the second ends of the arms being woven through the conical net proximate the opening, wherein the apex of the conical net is proximate the first end of the arms of the grasper (114).

4. The extraction device of claim 3, wherein the apex of the net is between the second end of the catheter (108) and the opening for receiving the foreign body.

5. The extraction device of any one of claims 2-4 wherein, for each arm of the grasper (114), the aperture is formed from bending the second end of the arm inward to form an eyelet, and/or wherein the first arm of the four arms of staggered length is the longest arm.

6. The extractor device according to claim 1, the plurality of arms being at least three arms, the second end of each arm comprising an aperture;
wherein the slidable coupling of the control fiber (120) to the second end of the first arm of the plurality of arms and to the second end of each subsequent arm of the plurality of arms and again to the second end of the first arm is achieved by the control fiber (120) passing through the corresponding aperture at the corresponding second end of the corresponding arm.

7. The extractor device (100) of claim 6, wherein, for each arm of the grasper (114), the aperture is an eyelet formed from bending the second end of the arm, preferably wherein, for each arm of the grasper (114), the eyelet is formed from bending the second end of the arm inward.

8. The extraction device of claim 6, wherein, for each arm of the grasper (114), the aperture is a hole passing through the second end of the arm.

9. The extraction device of any one of claims 6-8, the grasper (114) further comprising a conical net having an opening for receiving the foreign body and an apex, the loop of control fiber (120) at the second ends of the arms being woven through the conical net proximate the opening, wherein the apex of the conical net is proximate the first end of the arms of the grasper (114).

10. The extraction device of any one of claims 6-9, wherein the first arm of the at least three arms of staggered length is the longest arm, or wherein the first arm of the at least three arms of staggered length is the shortest arm.

11. The extractor device (100) of claim 1, wherein, for each arm of the plurality of arms, the second end comprises an aperture and the control fiber (120) is slideably coupled to the second end by passing through the aperture, and wherein the control fiber (120) passes through the aperture of the first arm twice.

12. The extractor device (100) of claim 11, wherein, for each arm of the grasper (114), the aperture is an eyelet formed from bending the second end of the arm, preferably wherein, for each arm of the grasper (114), the eyelet is formed from bending the second end of the arm inward.

13. The extraction device of claim 11, wherein, for each arm of the grasper (114), the aperture is a hole passing through the second end of the arm.

14. The extraction device of any one of claims 10-13, the grasper (114) further comprising a conical net having an opening for receiving the foreign body and an apex, the loop of control fiber (120) at the second ends of the arms being woven through the conical net proximate the opening, wherein the apex of the conical net is proximate the first end of the arms of the grasper (114).

15. The extraction device of any one of claims 10-14, wherein the first arm of the plurality of arms of staggered length is the longest arm, or wherein the first arm of the plurality of arms of staggered length is the shortest arm.

## Patentansprüche

1. Eine Extraktionsvorrichtung (100), umfassend:
einen Griff mit einem Antriebsschlitten (106), der beweglich mit einem Griffkörper gekoppelt ist, wobei der Antriebsschlitten (106) zwischen einer ausgefahrenen Position und einer eingefahrenen Position beweglich ist, wobei der Griff mit einem ersten Ende eines Katheters (108) gekoppelt ist, wobei der Antriebsschlitten (106) mit einem ersten Ende eines Antriebsdrahtes (300) innerhalb des Katheters (108) gekoppelt ist, wobei der Antriebsdraht (300) im Wesentlichen die gleiche Länge wie der Katheter (108) aufweist;
einen Greifer (114), der fest mit einem zweiten Ende des Antriebsdrahtes (300) distal zu dem ersten Ende des Antriebsdrahtes (300) gekoppelt ist, wobei der Greifer (114) eine Vielzahl von Armen mit gestaffelter Länge umfasst, wobei jeder Arm ein erstes Ende, das mit dem Antriebsdraht (300) gekoppelt ist, und ein zweites Ende distal zu dem Antriebsdraht (300) aufweist, wobei der Greifer (114) durch den Antriebsschlitten (106) über den Antriebsdraht (300) zwischen einer verstauten Position, in der sich der Greifer (114) innerhalb des Katheters (108) in der Nähe des zweiten Endes des Katheters (108) befindet, wobei die Arme des Greifers (114) nach innen eingeklappt sind und sich der Antriebsschlitten (106) in der zurückgezogenen Position befindet, und einer offenen Position, in der sich der Greifer (114) außerhalb des Katheters (108) befindet, wobei die Arme nach außen ausgefahren sind und sich der Antriebsschlitten (106) in der ausgefahrenen Position befindet, bewegbar ist;
einen Steuerring (118), der gleitend mit dem Griffkörper verbunden ist und eine neutrale Position in der Nähe des ersten Endes des Katheters (108) aufweist; und
eine Steuerfaser (120) mit einem ersten Ende und einem zweiten Ende, die jeweils mit dem Steuerring (118) verbunden sind, wobei die Steuerfaser (120) innerhalb des Katheters (108) von dem ersten Ende des Katheters (108) zu dem Greifer (114) hinunterläuft und verschiebbar mit dem zweiten Ende eines ersten Arms der Vielzahl von Armen verbunden ist, wobei die Faser auch gleitend mit dem zweiten Ende jedes nachfolgenden Arms der Vielzahl von Armen und wieder mit dem zweiten Ende des ersten Arms gekoppelt ist, so dass eine Schleife zwischen den Armen des Greifers (114) gebildet wird, wenn sich der Greifer (114) in der offenen Position befindet, wobei die Steuerfaser (120) danach innerhalb des Katheters (108) von dem zweiten Ende des Katheters (108) zu dem ersten Ende des Katheters (108) nach oben läuft;
wobei jeder Arm von einer Mittelachse des zweiten Endes des Antriebsdrahtes (300) nach außen gekrümmt und von dieser weg vorgespannt ist;
wobei das Ziehen des Steuerrings (118) weg von der neutralen Position, während sich der Greifer (114) in der offenen Position befindet, die Schleife verengt, die zweiten Enden der Arme in Richtung der Mittelachse ablenkt und den Greifer (114) in eine Fangposition bringt, wodurch ein Fremdkörper, der durch die Schleife aufgenommen wurde, während sich der Greifer (114) in der offenen Position befand, zwischen den Armen gefangen wird.

2. Die Extraktionsvorrichtung nach Anspruch 1, wobei die Vielzahl von Armen vier Arme sind und das zweite Ende jedes Arms eine Öffnung aufweist;
wobei die verschiebbare Verbindung der Steuerfaser (120) mit dem zweiten Ende des ersten Arms der Vielzahl von Armen und mit dem zweiten Ende jedes nachfolgenden Arms der Vielzahl von Armen und wiederum mit dem zweiten Ende des ersten Arms dadurch erreicht wird, dass die Steuerfaser (120) durch die entsprechende Öffnung am entsprechenden zweiten Ende des entsprechenden Arms hindurchgeht; und
wobei für jeden Arm des Greifers (114) die Öffnung eine Öse ist, die durch Biegen des zweiten Endes des Arms gebildet wird.

3. Die Extraktionsvorrichtung nach Anspruch 2, wobei der Greifer (114) ferner ein konisches Netz mit einer Öffnung zur Aufnahme des Fremdkörpers und einem Scheitelpunkt umfasst, wobei die Schleife der Steuerfaser (120) an den zweiten Enden der Arme durch das konische Netz in der Nähe der Öffnung gewebt ist, wobei der Scheitelpunkt des konischen Netzes in der Nähe des ersten Endes der Arme des Greifers (114) liegt.

4. Die Extraktionsvorrichtung nach Anspruch 3, wobei der Scheitelpunkt des Netzes zwischen dem zweiten Ende des Katheters (108) und der Öffnung zur Aufnahme des Fremdkörpers angeordnet ist.

5. Die Extraktionsvorrichtung nach einem der Ansprüche 2 bis 4, wobei für jeden Arm des Greifers (114) die Öffnung durch Biegen des zweiten Endes des Arms nach innen gebildet wird, um eine Öse zu bilden, und/oder wobei der erste Arm der vier Arme mit gestaffelter Länge der längste Arm ist.

6. Die Extraktionsvorrichtung nach Anspruch 1, wobei die Vielzahl von Armen mindestens drei Arme sind und das zweite Ende jedes Arms eine Öffnung aufweist;
wobei die verschiebbare Verbindung der Steuerfaser (120) mit dem zweiten Ende des ersten Arms der Vielzahl von Armen und mit dem zweiten Ende jedes nachfolgenden Arms der Vielzahl von Armen und wieder mit dem zweiten Ende des ersten Arms dadurch erreicht wird, dass die Steuerfaser (120) durch die entsprechende Öffnung am entsprechenden zweiten Ende des entsprechenden Arms hindurchgeht.

7. Die Extraktionsvorrichtung (100) nach Anspruch 6, wobei für jeden Arm des Greifers (114) die Öffnung eine Öse ist, die durch Biegen des zweiten Endes des Arms gebildet wird, vorzugsweise wobei für jeden Arm des Greifers (114) die Öse durch Biegen des zweiten Endes des Arms nach innen gebildet wird.

8. Die Extraktionsvorrichtung nach Anspruch 6, wobei für jeden Arm des Greifers (114) die Öffnung ein Loch ist, das durch das zweite Ende des Arms verläuft.

9. Die Extraktionsvorrichtung nach einem der Ansprüche 6 bis 8, wobei der Greifer (114) ferner ein konisches Netz mit einer Öffnung zur Aufnahme des Fremdkörpers und einem Scheitelpunkt aufweist, wobei die Schleife der Steuerfaser (120) an den zweiten Enden der Arme durch das konische Netz in der Nähe der Öffnung gewebt ist, wobei der Scheitelpunkt des konischen Netzes in der Nähe des ersten Endes der Arme des Greifers (114) liegt.

10. Die Extraktionsvorrichtung nach einem der Ansprüche 6 bis 9, wobei der erste Arm der mindestens drei Arme mit gestaffelter Länge der längste Arm ist, oder wobei der erste Arm der mindestens drei Arme mit gestaffelter Länge der kürzeste Arm ist.

11. Die Extraktionsvorrichtung (100) nach Anspruch 1, wobei für jeden Arm der Vielzahl von Armen das zweite Ende eine Öffnung aufweist und die Steuerfaser (120) verschiebbar mit dem zweiten Ende verbunden ist, indem sie durch die Öffnung hindurchgeht, und wobei die Steuerfaser (120) zweimal durch die Öffnung des ersten Arms hindurchgeht.

12. Die Extraktionsvorrichtung (100) nach Anspruch 11, wobei für jeden Arm des Greifers (114) die Öffnung eine Öse ist, die durch Biegen des zweiten Endes des Arms gebildet wird, vorzugsweise wobei für jeden Arm des Greifers (114) die Öse durch Biegen des zweiten Endes des Arms nach innen gebildet wird.

13. Die Extraktionsvorrichtung nach Anspruch 11, wobei für jeden Arm des Greifers (114) die Öffnung ein Loch ist, das durch das zweite Ende des Arms verläuft.

14. Die Extraktionsvorrichtung nach einem der Ansprüche 10 bis 13, wobei der Greifer (114) ferner ein konisches Netz mit einer Öffnung zur Aufnahme des Fremdkörpers und einem Scheitelpunkt aufweist, wobei die Schleife der Steuerfaser (120) an den zweiten Enden der Arme durch das konische Netz in der Nähe der Öffnung gewebt ist, wobei der Scheitelpunkt des konischen Netzes in der Nähe des ersten Endes der Arme des Greifers (114) liegt.

15. Die Extraktionsvorrichtung nach einem der Ansprüche 10 bis 14, wobei der erste Arm der Vielzahl von Armen mit gestaffelter Länge der längste Arm ist, oder wobei der erste Arm der Vielzahl von Armen mit gestaffelter Länge der kürzeste Arm ist.

## Revendications

1. Dispositif d'extraction (100), comprenant :
une poignée comprenant un coulisseau d'entraînement (106) couplé de manière mobile à un corps de poignée, le coulisseau d'entraînement (106) étant mobile entre une position déployée et une position rétractée, la poignée étant couplée à une première extrémité d'un cathéter (108), le coulisseau d'entraînement (106) étant couplé à une première extrémité d'un fil d'entraînement (300) à l'intérieur du cathéter (108), le fil d'entraînement (300) ayant une longueur sensiblement égale à celle du cathéter (108) ;
un élément de préhension (114) couplé de manière fixe à une seconde extrémité du fil d'entraînement (300) distale par rapport à la première extrémité du fil d'entraînement (300), l'élément de préhension (114) comprenant une pluralité de bras de longueurs décalées, chaque bras ayant une première extrémité couplée au fil d'entraînement (300) et une seconde extrémité distale par rapport au fil d'entraînement (300), l'élément de préhension (114) pouvant être déplacé par le coulisseau d'entraînement (106) par l'intermédiaire du fil d'entraînement (300) entre une position rangée dans laquelle l'élément de préhension (114) est situé à l'intérieur du cathéter (108) à proximité de la seconde extrémité du cathéter (108), les bras de l'élément de préhension (114) étant repliés vers l'intérieur et le coulisseau d'entraînement (106) étant dans la position rétractée, et une position ouverte dans laquelle l'élément de préhension (114) est situé à l'extérieur du cathéter (108), les bras étant déployés vers l'extérieur et le coulisseau d'entraînement (106) étant dans la position déployée ;
une bague de commande (118) couplée de manière coulissante au corps de poignée et ayant une position neutre à proximité de la première extrémité du cathéter (108) ; et
une fibre de commande (120) ayant une première extrémité et une seconde extrémité couplées chacune à la bague de commande (118), la fibre de commande (120), descendant à l'intérieur du cathéter (108) depuis la première extrémité du cathéter (108) jusqu'à l'élément de préhension (114), étant couplée de manière coulissante à la seconde extrémité d'un premier bras parmi la pluralité de bras, la fibre étant également couplée de manière coulissante à la seconde extrémité de chaque bras suivant parmi la pluralité de bras et de nouveau à la seconde extrémité du premier bras de sorte qu'une boucle se forme entre les bras de l'élément de préhension (114) lorsque l'élément de préhension (114) est dans la position ouverte, la fibre de commande (120) remontant ensuite à l'intérieur du cathéter (108) depuis la seconde extrémité du cathéter (108) jusqu'à la première extrémité du cathéter (108) ;
dans lequel chaque bras est incurvé vers l'extérieur à partir d'un axe central de la seconde extrémité du fil d'entraînement (300) et est sollicité à l'opposé de celui-ci ;
dans lequel le fait de tirer la bague de commande (118) à l'opposé de la position neutre, alors que l'élément de préhension (114) est dans la position ouverte, resserre la boucle, en déviant les secondes extrémités des bras vers l'axe central et en mettant l'élément de préhension (114) dans une position de capture, piégeant ainsi, entre les bras, un corps étranger reçu à travers la boucle alors que l'élément de préhension (114) était dans la position ouverte.

2. Dispositif d'extraction selon la revendication 1, la pluralité de bras étant constituée de quatre bras, la seconde extrémité de chaque bras comprenant une ouverture ;
dans lequel le couplage coulissant de la fibre de commande (120) à la seconde extrémité du premier bras parmi la pluralité de bras et à la seconde extrémité de chaque bras suivant parmi la pluralité de bras et de nouveau à la seconde extrémité du premier bras est réalisé par le passage de la fibre de commande (120) à travers l'ouverture correspondante à la seconde extrémité correspondante du bras correspondant ; et
dans lequel, pour chaque bras de l'élément de préhension (114), l'ouverture est un œillet formé en pliant la seconde extrémité du bras.

3. Dispositif d'extraction selon la revendication 2, l'élément de préhension (114) comprenant en outre un filet conique ayant une ouverture permettant de recevoir le corps étranger et un sommet, la boucle de fibre de commande (120) au niveau des secondes extrémités des bras étant tissée à travers le filet conique à proximité de l'ouverture, dans lequel le sommet du filet conique est à proximité de la première extrémité des bras de l'élément de préhension (114).

4. Dispositif d'extraction selon la revendication 3, dans lequel le sommet du filet se trouve entre la seconde extrémité du cathéter (108) et l'ouverture permettant de recevoir le corps étranger.

5. Dispositif d'extraction selon l'une quelconque des revendications 2 à 4, dans lequel, pour chaque bras de l'élément de préhension (114), l'ouverture se forme en pliant la seconde extrémité du bras vers l'intérieur pour former un œillet, et/ou dans lequel le premier bras parmi les quatre bras de longueurs décalées est le bras le plus long.

6. Dispositif d'extraction selon la revendication 1, la pluralité de bras étant constituée d'au moins trois bras, la seconde extrémité de chaque bras comprenant une ouverture ;
dans lequel le couplage coulissant de la fibre de commande (120) à la seconde extrémité du premier bras parmi la pluralité de bras et à la seconde extrémité de chaque bras suivant parmi la pluralité de bras et de nouveau à la seconde extrémité du premier bras est réalisé par le passage de la fibre de commande (120) à travers l'ouverture correspondante à la seconde extrémité correspondante du bras correspondant.

7. Dispositif d'extraction (100) selon la revendication 6, dans lequel, pour chaque bras de l'élément de préhension (114), l'ouverture est un œillet formé en pliant la seconde extrémité du bras, de préférence dans lequel, pour chaque bras de l'élément de préhension (114), l'œillet se forme en pliant la seconde extrémité du bras vers l'intérieur.

8. Dispositif d'extraction selon la revendication 6, dans lequel, pour chaque bras de l'élément de préhension (114), l'ouverture est un trou traversant la seconde extrémité du bras.

9. Dispositif d'extraction selon l'une quelconque des revendications 6 à 8, l'élément de préhension (114) comprenant en outre un filet conique ayant une ouverture permettant de recevoir le corps étranger et un sommet, la boucle de fibre de commande (120) au niveau des secondes extrémités des bras étant tissée à travers le filet conique à proximité de l'ouverture, dans lequel le sommet du filet conique est à proximité de la première extrémité des bras de l'élément de préhension (114).

10. Dispositif d'extraction selon l'une quelconque des revendications 6 à 9, dans lequel le premier bras parmi les au moins trois bras de longueurs décalées est le bras le plus long, ou dans lequel le premier bras parmi les au moins trois bras de longueurs décalées est le bras le plus court.

11. Dispositif d'extraction (100) selon la revendication 1, dans lequel, pour chaque bras parmi la pluralité de bras, la seconde extrémité comprend une ouverture et la fibre de commande (120) est couplée de manière coulissante à la seconde extrémité en traversant l'ouverture, et dans lequel la fibre de commande (120) traverse l'ouverture du premier bras deux fois.

12. Dispositif d'extraction (100) selon la revendication 11, dans lequel, pour chaque bras de l'élément de préhension (114), l'ouverture est un œillet formé en pliant la seconde extrémité du bras, de préférence dans lequel, pour chaque bras de l'élément de préhension (114), l'œillet se forme en pliant la seconde extrémité du bras vers l'intérieur.

13. Dispositif d'extraction selon la revendication 11, dans lequel, pour chaque bras de l'élément de préhension (114), l'ouverture est un trou traversant la seconde extrémité du bras.

14. Dispositif d'extraction selon l'une quelconque des revendications 10 à 13, l'élément de préhension (114) comprenant en outre un filet conique ayant une ouverture permettant de recevoir le corps étranger et un sommet, la boucle de fibre de commande (120) au niveau des secondes extrémités des bras étant tissée à travers le filet conique à proximité de l'ouverture, dans lequel le sommet du filet conique est à proximité de la première extrémité des bras de l'élément de préhension (114).

15. Dispositif d'extraction selon l'une quelconque des revendications 10 à 14, dans lequel le premier bras parmi la pluralité de bras de longueurs décalées est le bras le plus long, ou dans lequel le premier bras parmi la pluralité de bras de longueurs décalées est le bras le plus court.
